(19) Europäisches Patentamt

European Patent Office

Office européen des brevets

(11) EP 0 667 804 B1

## (12) EUROPEAN PATENT SPECIFICATION

(45) Date of publication and mention
of the grant of the patent:
**19.05.1999 Bulletin 1999/20**

(21) Application number: **94902190.1**

(22) Date of filing: **28.10.1993**

(51) Int. Cl.⁶: $B01J\ 8/26$, $C07C\ 17/10$, $C07C\ 17/02$, $C07C\ 19/00$, $C07C\ 19/045$, $C07C\ 29/124$, $C07C\ 31/04$, $C07C\ 31/20$

(86) International application number:
**PCT/US93/10376**

(87) International publication number:
**WO 94/09897 (11.05.1994 Gazette 1994/11)**

### (54) METHOD OF PREPARING ALKANOLS AND GLYCOLS

VERFAHREN ZUR HERSTELLUNG VON ALKANOLEN UND GLYKOLEN

PROCEDE DE PREPARATION D'ALCANOLS ET DE GLYCOLS

(84) Designated Contracting States:
**AT BE CH DE DK ES FR GB GR IE IT LI LU MC NL PT SE**

(30) Priority: **04.11.1992 US 971899**
**04.08.1993 US 101739**

(43) Date of publication of application:
**23.08.1995 Bulletin 1995/34**

(73) Proprietor: **ENERGIA ANDINA LTD**
**New York, NY 10016 (US)**

(72) Inventors:
• **MILLER, Jorge, P.**
**Santa Fe de Bogota (CO)**

• **KLING, Miguel**
**Santa Fe de Bogota (CO)**

(74) Representative:
**Bubb, Antony John Allen et al**
**GEE & CO.**
**Chancery House**
**Chancery Lane**
**London WC2A 1QU (GB)**

(56) References cited:
GB-A- 559 080              GB-A- 587 969
US-A- 3 172 915

• JOURNAL OF THE AMERICAN CHEMICAL SOCIETY vol. 107, no. 24 , 1985 , WASHINGTON, DC US pages 7097 - 7105 G A OLAH ET AL

**Description**

*Field of the Invention*

A lower alkane is reacted with a metal chloride to produce the corresponding alkyl chloride. Reacting the obtained alkyl chloride with magnesium oxide and steam yields the corresponding alkanol. In a similar fashion lower alkenes or lower alkanols are converted to corresponding glycols.

*Background*

Methane has previously been chlorinated with gaseous chlorine or subjected to oxychlorination with oxygen and hydrochloric acid to form methyl chloride together with other chlorides, such as dichloromethane, trichloromethane and carbon tetrachloride. In the halogenation of methane by either method, hydrochloric acid is produced. Such hydrochloric acid must be recovered, dehydrated by azeotropic distillation and recycled. Reduced chloromethanes are then hydrolyzed in vapor phase to methanol, formaldehyde, formic acid, carbon dioxide and hydrochloric acid. Resulting compositions depend on the chlorination selectivity to methyl chloride and to other chlorides. Corrosion and problems involved with handling chlorine and hydrochloric acid are substantial.

US-A-3,172,915 discloses the preparation of oxygenated methane derivatives, by converting methane to methanol dimethyl ether or both involving high temperatures and several separation and reaction steps.

*Summary of the Invention*

An object of the invention is to overcome or eliminate previously-encountered problems and to obtain a simplified process for converting an alkane to the corresponding alkanol. The method is based on the formation of an alkyl chloride and its hydration to the corresponding alcohol.

According to this process, methane (the preferred alkane) is reacted with a metal chloride (metallic chloride), wherein the metal is in the higher of two possible valence states, to form methyl chloride, the corresponding metal chloride (metallous chloride), wherein the metal is in the lower of two possible valence states, and hydrochloric acid. The obtained methyl chloride and hydrochloric acid are reacted with magnesium oxide to form methyl alcohol and magnesium chloride hydrate. The obtained metallous chloride is reacted with hydrochloric acid and oxygen to form metallic chloride, and the magnesium chloride hydrate is converted to magnesium oxide and hydrochloric acid. Lower alkenes are similarly converted to corresponding glycols.

According to one aspect of the present invention there is provided a process for producing a lower monobasic or dibasic alcohol having from 1 to 4 carbon atoms which comprises the following steps:

a) reacting a suitable starting material with a metallic halide, wherein the metal is in the higher of two possible valence states, to form a reaction product, a corresponding metallous halide having the metal in the lower of the two possible valence states, and hydrohalic acid, and

b) reacting said reaction product of step (a) and hydrohalic acid, with magnesium oxide to form the corresponding lower monobasic or dibasic alkanol and magnesium halide hydrate; and

where the starting material for forming a lower monobasic alcohol is a lower alkane having from 1 to 4 carbon atoms, from which the corresponding lower alkanol is obtained; and the starting material for forming a lower dibasic alcohol is either a lower alkanol having from 1 to 4 carbon atoms or a lower alkene having from 1 to 4 carbon atoms, from which the corresponding lower glycol is obtained.

Preferably, the above process further comprises:

c) reacting the metallous halide with hydrohalic acid and oxygen to form metallic halide; and

d) converting the magnesium halide hydrate to magnesium oxide and hydrohalic acid.

Conveniently, the metal halide is copper chloride.

In one embodiment the process is substantially conducted in a fluidized bed.

According to another aspect of the present invention there is provided a process for producing methanol from methane which comprises:

a) reacting methane with a metallic chloride, wherein the metal is in the higher of two possible valence states, to form methyl chloride, the corresponding metallous chloride having the metal in the lower of two possible valence states, and hydrochloric acid;

b) passing the methyl chloride and hydrochloric acid obtained from step (a), together with steam, through a magnesium zeolite catalyst to form methyl alcohol and hydrochloric acid;

c) reacting the methyl alcohol and hydrochloric acid obtained from step (b) with magnesium oxide to obtain methyl alcohol and magnesium chloride hydrate; and

d) converting the magnesium chloride hydrate to magnesium oxide and hydrochloric acid.

Preferably, the metal chloride is copper chloride. Conveniently, the process is substantially conducted in a fluidized bed.

Preferably, the process further comprises the steps of:

a) reacting methane with cupric chloride in a fluidized bed comprising a mixture of magnesium oxide, cupric chloride and cuprous chloride to form a combination of gases comprising hydrochloric acid, methyl chloride and unreacted methane;

b) passing the combination of gases and steam through a magnesium zeolite catalyst to obtain an admixture of methyl alcohol, hydrochloric acid and methane;

c) passing the admixture through a fluidized bed containing magnesium oxide, which adsorbs all of the hydrochloric acid;

d) condensing the methyl alcohol from the remaining combination of methyl alcohol and methane; and

e) recirculating the methane to step (a).

In one embodiment the fluidized bed comprises a mixture of cupric chloride, cuprous chloride and magnesium oxide, and the mixture comprises the stated components in the approximate molar ratio of 1/0.1/2 of cupric chloride/cuprous chloride/ magnesium oxide. This mixture may further comprise cupric oxide.

According to a further aspect of the present invention, there is provided an apparatus for producing methanol from methane which comprises:

a) a fluidized bed reactor comprising, as reactant, the fluidized bed of Claim 9, means for introducing methane into the reactor and into the fluidized bed therein, and conduit means to conduct unreacted methane and produced gases therefrom and to (b);

b) a catalyst-containing reactor, means to introduce steam into the conduit means, means to conduct the resulting admixture through catalyst therein, and second conduit means to conduct unreacted methane and reaction products therefrom and to (c);

c) a first fluidizer comprising adsorbent means to adsorb hydrochloric acid, and third conduit means to conduct remaining gaseous components from said fluidizer and to (d); and

d) condenser means to condense methanol, and fourth conduit means to return unreacted methane to fluidized bed reactor (a).

Preferably, the apparatus further comprises

e) a second fluidizer, means to conduct spent reactant from the fluidizer bed reactor to the second fluidizer, means to conduct air and hydrochloric acid into said second fluidizer and through the spent reactant therein to regenerate said spent reactant, means to conduct regenerated reactant to said fluidized bed reactor, and fifth conduit means to conduct gases from the second fluidizer to (f); and

f) a third fluidizer which comprises adsorbent means to adsorb all traces of hydrochloric acid in gases withdrawn from the second fluidizer, and means to exhaust clean gas.

In one embodiment, the apparatus further comprises:

g) a fourth fluidizer, means to conduct spent absorbent means from the third fluidizer to the fourth fluidizer, means to conduct air into the fourth fluidizer and through the spent absorbent means therein to regenerate said spent absorbent means, means for conveying regenerated absorbent means to the third fluidizer, and means to conduct effluent gases to the second fluidizer.

Preferably, the apparatus further comprises:

h) a fifth fluidizer, means to convey spent absorbent means from the first fluidizer to the fifth fluidizer, means to conduct air into said fifth fluidizer and through the spent absorbent means therein to regenerate said spent absorbent means, means to convey regenerated absorbent means to the first fluidizer, and means to conduct gases emanating therefrom to the second fluidizer.

According to a still further aspect of the present invention there is provided an apparatus for producing a lower monobasic or dibasic alcohol as defined above which comprises:

a) a first fluidized bed reactor, conduit means to introduce reactant into the first fluidized bed reactor, and conduit means to conduct gas from said first reactor to a first cyclone, conduit means to return solids from the first cyclone to the first reactor, and conduit means to conduct gas from the first cyclone to a second reactor;

b) conduit means to conduct steam into the second reactor, and conduit means to conduct reacted gases from the second reactor to a third reactor, which is a fluidized bed reactor;

c) conduit means to conduct gas from the third reactor to a second cyclone, conduit means to return solids from the second cyclone to the third reactor, means to conduct gas from the second cyclone to a condenser, and means to withdraw condensate from the condenser; and

d) conduit means to conduct gas from the condenser to compressor means to recirculate compressed gas to the conduit means to introduce reactant into the first fluidized bed reactor.

Preferably, the apparatus further comprises conduit means for conducting spent reactant from the first fluidized bed reactor to a fourth reactor, which is also a fluidized bed reactor, conduit means for introducing gas into the fourth reactor, and conduit means for conducting regenerated reagent from the fourth reactor back to said first fluidized bed reactor.

In one embodiment, the apparatus further comprises a fifth reactor, which is also a fluidized bed reactor, conduit means to conduct spent fluidized bed material from the third reactor to the fifth reactor, conduit means for introducing gas into the fifth reactor, and conduit means for conducting regenerated fluidized bed material back to said third reactor.

Brief Description of the Drawings

Figure 1 is a flow diagram depicting one embodiment of the claimed process.

Figure 2 is a flow diagram depicting a second and simplified embodiment of the claimed process.

Details

Methane is reacted with a metal chloride which is capable of chlorinating methane. The metal is one which concurrently reduces its valence to a lower state. For example, cupric chloride reacts with methane to form methyl chloride, cuprous chloride and hydrochloric acid, according to reaction (I)

$$2 \text{ CuCl}_2 + \text{CH}_4 \rightarrow 2 \text{ CuCl} + \text{CH}_3\text{Cl} + \text{HCl} \qquad \text{(I)}$$

The obtained methyl chloride and hydrochloric acid are next reacted with steam and a catalyst containing magnesium oxide, according to reaction scheme (II)

$$\text{H}_2\text{O} + \text{CH}_3\text{Cl} + \text{HCl} + \text{MgO} \rightarrow \text{CH}_3\text{OH} + \text{MgCl}_2 + \text{H}_2\text{O} \qquad \text{(II)}$$

Air and oxygen are passed countercurrent through the magnesium chloride to recover hydrochloric acid according to reaction scheme (III)

$$\text{MgCl}_2 \cdot \text{x H}_2\text{O} \rightarrow \text{MgO} + 2\text{HCl} \qquad \text{(III)}$$

and then through the cuprous chloride to reform cupric chloride according to reaction scheme (IV)

$$2\text{HCl} + 1/2 \text{ O}_2 + 2\text{CuCl} \rightarrow 2\text{CuCl}_2 + \text{H}_2\text{O} \qquad \text{(IV)}.$$

Reaction (I) is advantageously carried out at temperatures between 300°C and 360°C, at which temperatures there is no formation of chlorine by decomposition of cupric chloride. Such decomposition takes place at 993°C to yield chlorine. By keeping the temperature low, the possibility of overchlorination of the methyl chloride to higher chlorides is minimized.

Reaction (II) is advantageously conducted at 200°C or less in order to avoid adsorbing chlorides and to release them according to the law of mass action.

Reaction (III) is advantageously carried out at about 200°C, and reaction (IV) is advantageously carried out within the approximate range of from 300°C to 380°C.

The preferred method is a continuous process, using fluidized-bed reactors. However, fluidized bed reactors are not necessary, and batch reactions can be employed. Instead of a metal chloride, such as copper chloride, in reaction (I), a mixture can be employed. The preferred mixture is one of cupric chloride, cuprous chloride and magnesium oxide. This particular mixture is preferably used for chlorination of methane because, by diluting the copper chloride

4

with magnesium oxide, less higher methyl chlorides are formed. Also, when reoxidizing in the presence of hydrochloric acid, the magnesium chloride formed reacts with any copper oxide formed to produce copper chloride. Magnesium oxide also serves to increase porosity.

$$MgCl_2 + CuO \rightarrow MgO + CuCl_2 \qquad\qquad (V)$$

Excess cuprous chloride adsorbs any formed chlorine.

$$CuCl + 1/2\ Cl_2 \rightarrow CuCl_2 \qquad\qquad (VI)$$

Instead of reacting methyl chloride with magnesium oxide in reaction (II), a magnesium zeolite can be used to hydrolyze the methyl chloride to methanol and hydrochloric acid; at 200°C the hydrochloric acid is next adsorbed by magnesium oxide. At temperatures in excess of 115°C, $MgCl_2 \cdot 4H_2O$ is formed, completely adsorbing all of the hydrochloric acid, which can be recovered by heating to 200°C, while passing air therethrough.

The mechanism and kinetics of the thermal decomposition of magnesium chloride hydrates have been reported (Kirk-Othmer Encyclopedia of Chemical Technology, Vol. 14-623, Third Edition). The reactions which are reversible take place in stages as shown.

$$95°C - 115°C\ MgCl_2 \cdot 6\ H_2O \leftrightharpoons MgCl_2 \cdot 4\ H_2O + 2\ H_2O$$

$$135°C - 180°C\ MgCl_2 \cdot 4\ H_2O \leftrightharpoons Mg(OH) + HCl + 3\ H_2O$$

$$186°C - 230°C\ MgCl_2 \cdot H_2O \leftrightharpoons Mg(OH)Cl + HCl$$

$$230°C - Mg(OH)Cl \leftrightharpoons MgO + HCl$$

Advantage is taken of these properties of magnesium chloride hydrates to adsorb and recover hydrochloric acid.

The exceedingly high conversion of methyl chloride to methyl alcohol (practically 100%), by the magnesium form of the zeolite (Mg $Z_2$), can be attributed to the following reactions:

$$MgZ_2 + 2\ CH_3Cl \rightarrow MgCl_2 + 2\ CH_3Z$$

$$CH_3Z + H_2O \rightarrow HZ + CH_3OH$$

and

$$MgCl_2 + H_2O \rightarrow Mg(OH)_2 + 2\ HCl$$

$$Mg(OH)_2 + 2\ HZ \rightarrow MgZ_2 + 2\ H_2O$$

In this case the magnesium zeolite acts like a catalyst.

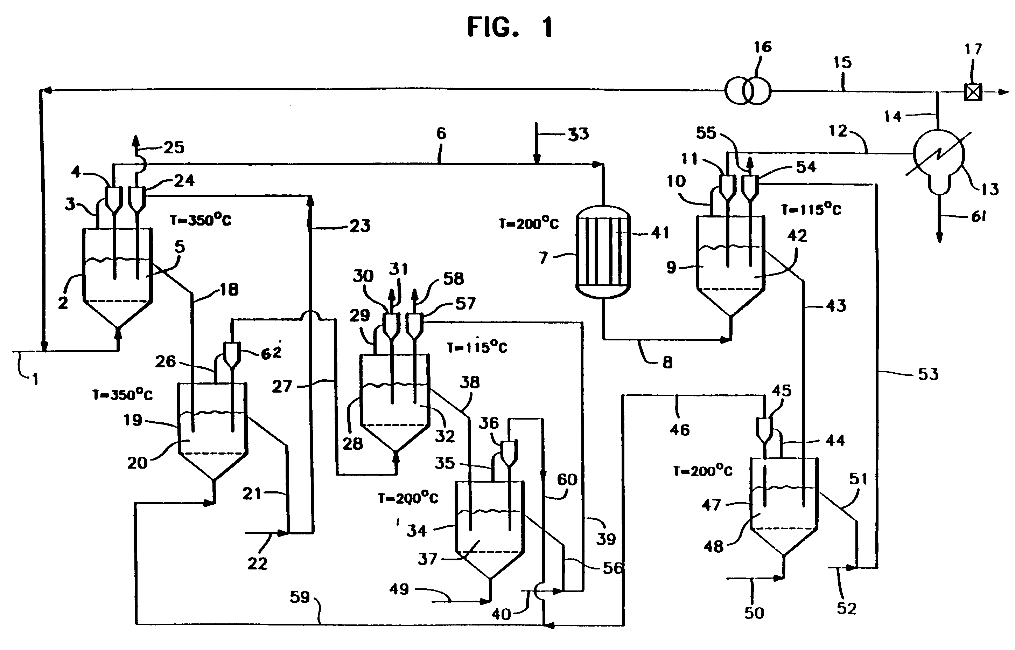

With reference to Figure 1, which depicts a typical continuous process using fluidized reactors, methane is introduced to fluid bed fluidizer 2 through line 1, where it reacts with cupric chloride contained in fluidized reactant 5, composed of a mixture of magnesium oxide, cupric chloride and cuprous chloride. (Alternatively, cupric bromide and cuprous bromide can be used). The reacted gas, comprising mostly hydrochloric acid, methyl chloride and excess methane, flows through line 3 to cyclone 4, which returns dust to reactor 2. Gas, leaving cyclone 4 through line 6, enters reactor 7, which contains catalyst (magnesium zeolite) 41, together with steam provided through line 33. Reacted gases, comprising methyl alcohol, hydrochloric acid and excess methane, leave reactor 7 through line 8, which delivers them to fluidizer 9, containing magnesium oxide 42, which adsorbs all the hydrochloric acid.

Gases leaving fluidizer 9 through line 10 to cyclone 11, which returns dust to fluidizer 9, contain methyl alcohol and excess methane. These gases are led through line 12 to condenser 13, where methyl alcohol is condensed and leaves condenser 13 through line 61.

Non-condensed methane leaves condenser 13 through line 14 to bleed valve 17 and through line 15 to compressor 16, which recirculates excess methane to line 1.

Spent reactant 5 from fluidizer 2 flows through line 18 to fluidizer 19, where it meets a flow of gas containing air and hydrochloric acid; cuprous chloride therein is regenerated back to cupric form.

Regenerated reagent 20 flows through line 21, where it meets conveying gas air 22, which lifts it through line 23 to cyclone 24, where conveying gas (air) is exhausted through line 25 to the atmosphere and reagent 5 is delivered by cyclone 24 to fluidizer 2.

Gases from fluidizer 19, containing possible traces of hydrochloric acid, are led through line 26 to cyclone 62, returning dust to fluidizer 19 and delivering gases through line 27 to fluidizer 28, which contains magnesium oxide 32, which adsorbs all traces of hydrochloric acid. The purified gas is bled to the atmosphere through line 29 and cyclone 30, which returns dust to fluidizer 28 and exhausts clean gas, free of pollution, through line 31.

Spent magnesium oxide 32 leaves fluidizer 28 through line 38, which delivers it to fluidizer 34, where it meets a flow of air, which regenerates the spent magnesium oxide 37. Regenerated magnesium oxide is conducted through line 56, where a conveying gas 40 lifts it through line 39 to cyclone 57. Conveying gas is exhausted through line 58 to the atmosphere, and regenerated magnesium oxide is delivered to fluidizer 28. Gases leaving fluidizer 34, containing air and hydrochloric acid, are led through line 35 to cyclone 36, where dust is returned to fluidizer 34, and gases are led through line 60 to line 59.

Spent magnesium oxide 42 leaves fluidizer 9 through line 43, which delivers it to fluidizer 47, where it meets a flow of air, introduced through line 50, which regenerates the spent magnesium oxide 48. Regenerated magnesium oxide flows through line 51, where it meets a conveying gas (air) 52, which lifts it to cyclone 54 through line 53. Conveying gas is exhausted through line 55, and cyclone 54 delivers regenerated magnesium oxide to fluidizer 9.

Gases leaving fluidizer 47, containing hydrochloric acid and air, are delivered through line 44 to cyclone 45, where dust is returned to fluidizer 47, and gases are led through line 46 to line 59 and, together with gases from line 60, enter fluidizer 19.

Air enters fluidizer 34 through line 49; air enters fluidizer 47 through line 50.

Temperatures indicated in Figure 1 are indicative. Reactant 5 is made, e.g., by mixing cuprous chloride, cupric chloride and magnesium oxide, the molar proportions suggested are:

| cupric chloride | 1 mole |
|---|---|
| cuprous chloride | 0.1 mole |
| magnesium oxide | 2 moles |

The reagent is advantageously made as follows:

1.1 mole of cupric chloride is dissolved in water to saturation. 2 moles of magnesium oxide are added. The mixture is evaporated to dryness and granulated.

The granulated product is then reduced with methane or hydrogen until 0.1 mole of copper chloride is reduced to cuprous chloride. When regenerating the reagent, cuprous chloride must always be present.

Magnesium oxide serves to tone down the activity of the cupric chloride. Other diluent materials can be used in combination with magnesium oxide (aluminum oxide, silica, fullers earth, etc.).

When conversion per pass is limited to less than 20%, overchlorination of the methane is limited to less than 1%. Increasing magnesium oxide in the reagent also has the same effect.

Magnesium zeolite catalyst is preferably prepared as follows: Type A or Type X zeolite, as defined in "Kirk-Othmer Encyclopedia of Chemical Technology", 3d Edition, Vol. 15, Page 665, is placed in a column, and a solution of soluble magnesium salt (sulfate, nitrate, etc.) is passed through the zeolite, whereby sodium is exchanged for magnesium. The zeolite in the magnesium form is then washed and dried, ready for use. The process is well known ("Kirk-Othmer Encyclopedia of Chemical Technology", 3d Edition, Vol. 13, page 678, etc.).

Although the preceding illustration has been made with copper chlorides, such chlorides are optionally replaced with bromides. Also, methane is optionally replaced with ethane, propane or n-butane to produce corresponding alcohols.

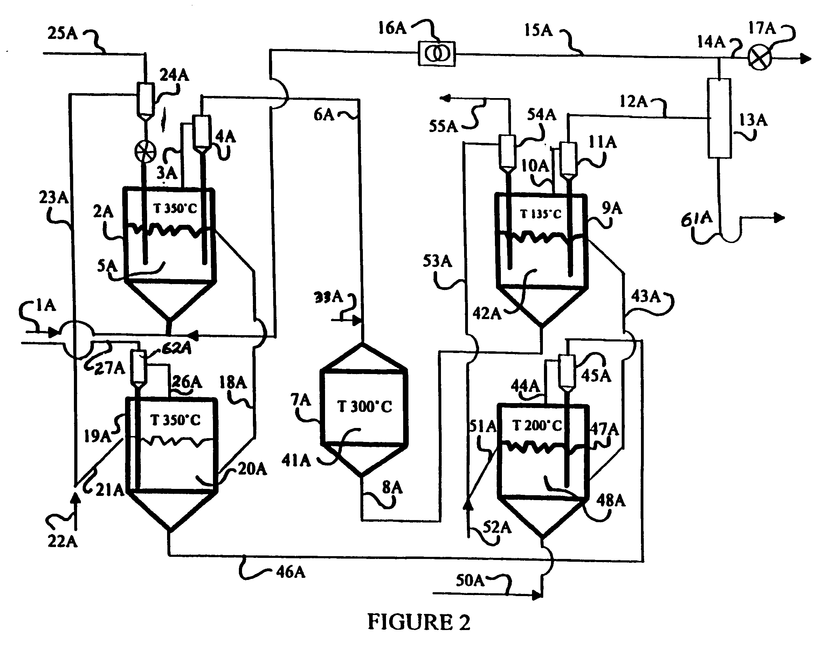

An alternative embodiment (Figure 2) omits fluidizer 28 and fluidizer 34 of Figure 1. In Figure 2 corresponding equipment is designated by similar numbers with an A suffix for ease of comparison. All of the hydrochloric acid from fluidizer 47A is completely absorbed in reactor 19A without forming chlorine.

A careful thermodynamic analysis of the reactions involves the following:

REACTION CONSTANTS (T=300K°)

$$2HCl+1/2\ O_2 \rightarrow Cl_2+H_2O \qquad \Delta F=-9080\ cal \qquad K=9.35x10^6$$
$$2CuCl=1/2\ O_2 \rightarrow CuCl_2+CuO \qquad \Delta F=-15700\ cal \qquad K=3.01x10^{11}$$
$$CuO+HCl \rightarrow CuCl_2+H_2O \qquad \Delta F=-42857\ cal \qquad K=2.16x10^{31}$$

The ease of reaction depends on the reaction constant K; thus, long before any chlorine is formed from the oxidation of the hydrochloric acid, it reacts with the copper oxide present.

In order to insure this, an excess of copper oxide is incorporated in the original reaction mixture. The preferred composition of reactant 5 contains at least 0.1 mole of copper oxide in addition to magnesium oxide and cuprous chloride, e.g.,

| | |
|---|---|
| cupric chloride | 1 mole |
| cupric oxide | 0.1 mole |
| cuprous chloride | 0.1 mole |
| magnesium oxide | 2 moles |

Figure 2 illustrates a simplified configuration of Figure 1, wherein a lower alkane is converted to a corresponding lower alkanol in a manner corresponding to that disclosed with regard to Figure 1, but with the inclusion of metallic oxide, e.g. cupric oxide, in reactant 5.

When a lower alkene, e.g. ethylene, is processed in the same equipment under corresponding conditions, it is first chlorinated to 1,2-dichloroethane and hydrolyzed to ethylene glycol. The only difference is that a larger proportion of steam is required to prevent its condensation in reactor 7A along with a higher temperature in fluidizer 9A (135°C). The following data show the vapor pressures versus temperatures for ethylene glycol.

| Vapor Pressure (mm Hg) | Temperature (°C) |
|---|---|
| 10 | 92.1 |
| 20 | 105.8 |
| 40 | 120.0 |
| 60 | 129.5 |
| 100 | 141.8 |
| 200 | 158.5 |
| 400 | 178.5 |
| 760 | 197.3 |

Thus, if the partial pressure of ethylene glycol is 80 mm Hg, in view of excess steam and excess ethylene, the temperature in fluidizer 9A can be held at 135°C.

When ethyl alcohol is vaporized and similarly processed in the same equipment, it is first chlorinated to ethylene chlorohydrin, which is hydrolyzed in turn to ethylene glycol. The same temperature precautions are observed.

With reference to Figure 2, which depicts a typical continuous process using fluidized reactors, ethylene is introduced to fluidized bed reactor 2A through line 1A, where it reacts with cupric chloride contained in fluidized reactant 5A, composed of a mixture of cupric chloride, cupric oxide, magnesium oxide, and cuprous chloride. Alternatively, bromides are used instead of chlorides.

The reacted gas, comprised mostly of hydrochloric acid, 1,2-dichloroethane and excess ethylene, flows through line 3A to cyclone 4A, which returns dust to reactor 2A. Gas, leaving cyclone 4A through line 6A, enters reactor 7A, which contains catalyst 41A (magnesium zeolite), together with steam provided through line 33A. Reacted gases, comprising ethylene glycol, hydrochloric acid and excess methane, leave reactor 7A through line 8A, which delivers them to fluidizer 9A, containing magnesium oxide 42A, which absorbs all of the hydrochloric acid. Gases leaving fluidizer 9A through line 10A to cyclone 11A, which returns dust to fluidizer 9A, contain ethylene glycol, excess ethylene, and water vapor. These gases are led through line 12A to condenser 13A, where glycol and water vapor are condensed, and leave condenser 13A through line 61A.

Non-condensed ethylene leaves condenser 13A through line 14A to bleed valve 17A and through line 15A to compressor 16A, which recirculates excess ethylene to line 1A.

Spent reactant 5A from fluidizer 2A flows through line 18A to fluidizer 19A, where it meets a flow of gas containing air and hydrochloric acid; cuprous chloride therein is regenerated back to cupric form.

Regenerated reagent 20A flows through line 21A, where it meets conveying gas (air) 22A, which lifts it through line 23A to cyclone 24A, wherein conveying gas (air) is exhausted through line 25A to the atmosphere, and reagent 5A is delivered by cyclone 24A to fluidizer 2A.

Spent magnesium oxide 42A leaves fluidizer 9A through line 43A, which delivers it to fluidizer 47A, where it meets a flow of air introduced through line 50A, which regenerates the spent magnesium oxide 48A. Regenerated magnesium oxide flows through line 51A, where it meets a conveying gas (air) 52A, which lifts it to cyclone 54A through line 53A. Conveying gas is exhausted through line 55A, and cyclone 54A delivers regenerated magnesium oxide to fluidizer 9A.

Gases leaving fluidizer 47A, containing hydrochloric acid and air, are delivered through line 44A to cyclone 45A, where dust is returned to fluidizer 47A, and gases are led through line 46A to fluidizer 19A.

Air enters fluidizer 47A through line 50A. Temperatures indicated in Figure 2 are indicative. Reactant 5A contains an excess of copper oxide.

Propylene glycol is similarly produced from propylene.

When ethyl alcohol, instead of ethylene, is the starting material for producing ethylene glycol, the condensed alcohol, water vapor, and glycol obtained in condenser 13A are sent to a splitter (not shown), where excess ethanol is separated from the glycol, and returned, vaporized, to line 1A.

The process and equipment disclosed are thus advantageously useful for converting lower alkanes to corresponding lower alkanols and for converting lower alkenes or lower alkanols to corresponding glycols. Lower monobasic or dibasic alcohols are produced by the following steps:

a) reacting a starting material with a metallic halide (wherein the metal is in the higher of two possible valence states) to obtain a reaction product, a corresponding metallous halide (wherein the metal is in the lower of the two possible valence states) and hydrohalic acid, and
b) reacting the reaction product of step (a) and hydrohalic acid with magnesium oxide to form the corresponding lower monobasic or dibasic alkanol;
wherein the starting material for forming a lower monobasic alcohol is a lower alkane, from which the corresponding lower alkanol is obtained; and the starting material for forming a lower dibasic alcohol is either a lower alkanol or a lower alkene, from which the corresponding lower glycol is obtained. Two continuous fluidized-bed systems are provided for conducting the necessary reactions.

The invention and its advantages are readily understood from the preceding description. It is apparent that various changes may be made in the process, in the system and in the compositions, without departing from the scope of the invention or sacrificing its material advantages. The process, systems and products hereinbefore described are merely illustrative of preferred embodiments of the invention.

## Claims

1.   A process for producing a lower monobasic or dibasic alcohol having from 1 to 4 carbon atoms which comprises the following steps:

a) reacting a suitable starting material with a metallic halide, wherein the metal is in the higher of two possible valence states, to form a reaction product, a corresponding metallous halide having the metal in the lower of the two possible valence states, and hydrohalic acid, and
b) reacting said reaction product of step (a) and hydrohalic acid, with magnesium oxide to form the corresponding lower monobasic or dibasic alkanol and magnesium halide hydrate; and
wherein the starting material for forming a lower monobasic alcohol is a lower alkane having from 1 to 4 carbon atoms, from which the corresponding lower alkanol is obtained; and the starting material for forming a lower dibasic alcohol is either a lower alkanol having from 1 to 4 carbon atoms or a lower alkene having from 1 to 4 carbon atoms, from which the corresponding lower glycol is obtained.

2. A process of Claim 1 which further comprises:

   c) reacting the metallous halide with hydrohalic acid and oxygen to form metallic halide; and
   d) converting the magnesium halide hydrate to magnesium oxide and hydrohalic acid.

3. A process of Claim 1 or 2 wherein the metal halide is copper chloride.

4. A process of any preceding claim substantially conducted in a fluidized bed.

5. A process for producing methanol from methane which comprises:

   a) reacting methane with a metallic chloride, wherein the metal is in the higher of two possible valence states, to form methyl chloride, the corresponding metallous chloride having the metal in the lower of two possible valence states, and hydrochloric acid;
   b) passing the methyl chloride and hydrochloric acid obtained from step (a), together with steam, through a magnesium zeolite catalyst to form methyl alcohol and hydrochloric acid;
   c) reacting the methyl alcohol and hydrochloric acid obtained from step (b) with magnesium oxide to obtain methyl alcohol and magnesium chloride hydrate; and
   d) converting the magnesium chloride hydrate to magnesium oxide and hydrochloric acid.

6. A process of Claim 5 wherein the metal chloride is copper chloride.

7. A process of Claim 5 or 6 substantially conducted in a fluidized bed.

8. A process according to any of Claims 5 to 7 which further comprises the steps of:

   a) reacting methane with cupric chloride in a fluidized bed comprising a mixture of magnesium oxide, cupric chloride and cuprous chloride to form a combination of gases comprising hydrochloric acid, methyl chloride and unreacted methane;
   b) passing the combination of gases and steam through a magnesium zeolite catalyst to obtain an admixture of methyl alcohol, hydrochloric acid and methane;
   c) passing the admixture through a fluidized bed containing magnesium oxide, which adsorbs all of the hydrochloric acid;
   d) condensing the methyl alcohol from the remaining combination of methyl alcohol and methane; and
   e) recirculating the methane to step (a).

9. A process of Claim 4, 7 or 8 wherein the fluidized bed comprises a mixture of cupric chloride, cuprous chloride and magnesium oxide.

10. A process of Claim 9 wherein the mixture comprises the stated components in the approximate molar ratio of 1/0.1/2 of cupric chloride/cuprous chloride/ magnesium oxide.

11. A process of Claim 9 or 10 wherein the mixture further comprises cupric oxide.

12. An apparatus for producing methanol from methane as defined in Claims 6 to 8 and 9 to 11 which comprises:

   a) a fluidized bed reactor comprising, as reactant, the fluidized bed of Claim 9, means for introducing methane into the reactor and into the fluidized bed therein, and conduit means to conduct unreacted methane and produced gases therefrom and to (b);
   b) a catalyst-containing reactor, means to introduce steam into the conduit means, means to conduct the resulting admixture through catalyst therein, and second conduit means to conduct unreacted methane and reaction products therefrom and to (c);
   c) a first fluidizer comprising adsorbent means to adsorb hydrochloric acid, and third conduit means to conduct remaining gaseous components from said fluidizer and to (d); and
   d) condenser means to condense methanol, and fourth conduit means to return unreacted methane to fluidized bed reactor (a).

13. An apparatus of Claim 12, further comprising:

e) a second fluidizer, means to conduct spent reactant from the fluidizer bed reactor to the second fluidizer, means to conduct air and hydrochloric acid into said second fluidizer and through the spent reactant therein to regenerate said spent reactant, means to conduct regenerated reactant to said fluidized bed reactor, and fifth conduit means to conduct gases from the second fluidizer to (f); and

f) a third fluidizer which comprises adsorbent means to adsorb all traces of hydrochloric acid in gases withdrawn from the second fluidizer, and means to exhaust clean gas.

**14.** An apparatus of Claim 12 or 13 further comprising:

g) a fourth fluidizer, means to conduct spent absorbent means from the third fluidizer to the fourth fluidizer, means to conduct air into the fourth fluidizer and through the spent adsorbent means therein to regenerate said spent absorbent means, means for conveying regenerated adsorbent means to the third fluidizer, and means to conduct effluent gases to the second fluidizer.

**15.** An apparatus of Claims 12 to 14 further comprising:

h) a fifth fluidizer, means to convey spent adsorbent means from the first fluidizer to the fifth fluidizer, means to conduct air into said fifth fluidizer and through the spent adsorbent means therein to regenerate said spent adsorbent means, means to convey regenerated adsorbent means to the first fluidizer, and means to conduct gases emanating therefrom to the second fluidizer.

**16.** An apparatus for producing a lower monobasic or dibasic alcohol as defined in Claims 1 to 5 and 9 to 11 which comprises:

a) a first fluidized bed reactor, conduit means to introduce reactant into the first fluidized bed reactor, conduit means to conduct gas from said first reactor to a first cyclone, conduit means to return solids from the first cyclone to the first reactor, and conduit means to conduct gas from the first cyclone to a second reactor;
b) conduit means to conduct steam into the second reactor, and conduit means to conduct reacted gases from the second reactor to a third reactor, which is a fluidized bed reactor;
c) conduit means to conduct gas from the third reactor to a second cyclone, conduit means to return solids from the second cyclone to the third reactor, means to conduct gas from the second cyclone to a condenser, and means to withdraw condensate from the condenser; and
d) conduit means to conduct gas from the condenser to compressor means to recirculate compressed gas to the conduit means to introduce reactant into the first fluidized bed reactor.

**17.** An apparatus of Claim 16 which further comprises conduit means for conducting spent reactant from the first fluidized bed reactor to a fourth reactor, which is also a fluidized bed reactor, conduit means for introducing gas into the fourth reactor, and conduit means for conducting regenerated reagent from the fourth reactor back to said first fluidized bed reactor.

**18.** An apparatus of Claim 16 or 17 which further comprises a fifth reactor, which is also a fluidized bed reactor, conduit means to conduct spent fluidized bed material from the third reactor to the fifth reactor, conduit means for introducing gas into the fifth reactor, and conduit means for conducting regenerated fluidized bed material back to said third reactor.

**19.** An apparatus of any of Claims 16 to 18 wherein:

the first fluidized bed reactor contains the fluidized bed of any of Claims 9 to 11.

## Patentansprüche

**1.** Verfahren zur Herstellung von niederen einwertigen oder zweiwertigen Alkoholen mit 1-4 Kohlenstoffatomen, welches die folgenden Schritte umfaßt:

a) Umsetzen eines geeigneten Ausgangsmaterials mit einem Metallhalogenid, worin das Metall sich in der höheren von zwei Oxidationsstufen befindet, unter Bildung eines Reaktionsproduktes, einem entsprechenden Metallhalogenid, worin das Metall in der niederen von zwei möglichen Oxidationsstufen ist und Halogenwasserstoffsäure, und

b) Umsetzen des Reaktionsprodukts gemäß Stufe a) und Halogenwasserstoffsäure mit Magnesiumoxid unter Bildung des entsprechenden niedereren einwertigen oder zweiwertigen Alkanols und Magnesiumhalogenidhydrat; und

worin das Ausgangsmaterial zur Bildung von niederen einwertigen Alkoholen ein niederes Alkan mit 1-4 C-Atomen ist, aus denen das entsprechende niedere Alkanol erhalten wird; und das Startmaterial zur Bildung eines niederen zweiwertigen Alkohols entweder ein niederes Alkanol mit 1-4 Kohlenstoffatomen oder ein niederes Alken mit 1-4 Kohlenstoffatomen ist, aus welchem das entsprechende niedere Glycol erhalten wird.

2. Verfahren gemäß Anspruch 1, welches weiterhin umfaßt:

c) Umsetzen des Metallhalogenids mit Halogenwasserstoffsäure und Sauerstoff zur Bildung von Metallhalogenid; und

d) Umsetzen des Magesiumhalogenidhydrats in Magnesiumoxid und Halogenwasserstoffsäure.

3. Verfahren gemäß Anspruch 1 oder 2, wobei das Metallhalogenid Kupferhalogenid ist.

4. Verfahren gemäß einem der vorstehenden Ansprüche, welches im wesentlichen in einem Fließbett durchgeführt wird.

5. Verfahren zur Herstellung von Methanol aus Methan, welches umfaßt:

a) Umsetzen von Methan mit Metallchlorid, worin das Metall sich in der höheren von zwei möglichen Oxidationsstufen befindet, unter Bildung von Methylchlorid, dem korrespondierenden Metallchlorid, worin das Metall in der niederen von zwei Oxidationsstufen vorliegt, und Salzsäure;

b) Durchleiten des Methylchlorids und der Salzsäure, welche in Stufe a) erhalten wurden, zusammen mit Wasserdampf durch einen Magnesium-Zeolit-Katalysator unter Bildung von Methanol und Salzsäure;

c) Umsetzen des Methanols und der Salzsäure, welche in Stufe b) erhalten wurden, mit Magnesiumoxid, unter Bildung von Methanol und Magnesiumchloridhydrat und

d) Umsetzen des Magnesiumchloridhydrats zu Magnesiumoxid und Salzsäure.

6. Verfahren gemäß Anspruch 5, worin das Metallchlorid Kupferchlorid ist.

7. Verfahren gemäß Anspruch 5 oder 6, welches im wesentlichen in einem Fließbett durchgeführt wird.

8. Verfahren gemäß einem der Ansprüche 5 bis 7, welches weiterhin die Schritte umfaßt:

a) Umsetzen von Methan mit Kupfer(II)chlorid in einem Fließbett, welches eine Mischung aus Magnesiumoxid, Kupfer(II)chlorid und Kupfer(I)chlorid enthält unter Bildung einer Mischung von Gasen, welche Salzsäure, Methylchlorid und nicht umgesetztes Methan enthält;

b) Durchleiten der Mischung der Gase mit Dampf durch einen Magnesium-Zeolit-Katalysator, um eine Mischung von Methanol, Salzsäure und Methan zu erhalten;

c) Durchleiten der Mischung durch ein Fließbett, welches Magnesiumoxid enthält, welches die gesamte Salzsäure adsorbiert;

d) Kondensieren des Methanols aus der zurückbleibenden Mischung aus Methanol und Methan und

e) Rezirkulieren des Methans in die Stufe a.

9. Verfahren gemäß Anspruch 4, 7 oder 8, wobei das Fließbett eine Mischung aus Kupfer(II)chlorid, Kupfer(I)chlorid und Magnesiumoxid enthält.

10. Verfahren gemäß Anspruch 9, wobei die Mischung die genannten Komponenten in ungefähr molaren Verhältnissen von 1 : 0,1 : 2 von Kupfer(II)chlorid : zu Kupfer(I)chlorid : Magnesiumoxid enthält.

11. Verfahren gemäß Anspruch 9 oder 10, wobei die Mischung weiterhin Kupfer(II)oxid enthält.

12. Eine Vorrichtung zur Herstellung von Methanol aus Methan gemäß den Ansprüchen 6 bis 8 und 9 bis 11, welche umfaßt:

a) einen Fließbettreaktor umfassend als Reaktand das Fließbett gemäß Anspruch 9, Vorrichtungen zum Einleiten von Methan in den Reaktor und in das darin befindliche Fließbett und Leitungsvorrichtungen, um nicht umgesetztes Methan und die gebildeten Gase abzuleiten und in b) einzuleiten;

b) einen Katalysator enthaltenden Reaktor, Vorrichtungen zum Einleiten von Dampf in die Leitungsvorrichtungen, Vorrichtungen zum Durchleiten der erhaltenen Mischung durch den darin enthaltenen Katalysator und zweite Leitungsvorrichtungen um nicht umgesetztes Methan und die Reaktionsprodukten abzuleiten und in c) einzuleiten;

c) ein erstes Fließbett, welches Adsorptionsmittel enthält, um Salzsäure zu adsorbieren und dritte Leitungsvorrichtungen zum Ableiten der verbleibenden gasförmigen Komponenten aus dem Fließbett und Einleiten in d); und

d) Kondensationsvorrichtungen zum Kondensieren von Methanol und vierte Leitungsvorrichtungen zum Zurückführen von nicht umgesetztem Methan in den Fließbettreaktor a).

13. Eine Vorrichtung gemäß Anspruch 12, welche weiterhin umfaßt:

e) ein zweites Fließbett, Vorrichtungen zum Ableiten von verbrauchtem Reaktand aus dem Fließbettreaktor in das zweite Fließbett, Vorrichtungen zum Einleiten von Luft und Salzsäure in das zweite Fließbett und durch den darin enthaltenen verbrauchten Reaktanden um den verbrauchten Reaktanden zu regenerieren, Vorrichtungen zum Einleiten des regenerierten Reaktanden in besagten Fließbettreaktor und fünfte Leitungsvorrichtungen zum Ableiten von Gasen aus dem zweiten Fließbett in f); und

f) ein drittes Fließbett, welches Adsorbtionsmittel enthält, zum Adsorbieren aller Spuren von Salzsäure in den Gasen, die aus dem zweiten Fließbett abgezogen werden und Vorrichtungen zum Ausstoßen des reinen Gases.

14. Vorrichtung gemäß Anspruch 12 oder 13, welche weiterhin umfaßt:

g) ein viertes Fließbett, Vorrichtungen zum Leiten von verbrauchten Adsorbtionsmitteln aus dem dritten Fließbett in das vierte Fließbett, Vorrichtungen zum Einleiten von Luft in das vierte Fließbett und durch die verbrauchten Adsorbtionsmittel, um die verbrauchten Adsorbtionsmittel zu regenerieren, Vorrichtungen zum Überführen von regenerierten Adsorbtionsmitteln in das dritte Fließbett und Vorrichtungen zum Leiten von entweichenden Gasen in das zweite Fließbett.

15. Eine Vorrichtung gemäß Anspruch 12 bis 14, welche weiterhin umfaßt:

h) ein fünftes Fließbett, Vorrichtungen zum Überführen von verbrauchten Adsorbtionsmittel aus dem ersten Fließbett in das fünfte Fließbett, Vorrichtungen zum Einleiten von Luft in das fünfte Fließbett und durch die darin enthaltenen verbrauchten Adsorbtionsmittel, um die verbrauchten Adsorbtionsmittel zu regenerieren, Vorrichtungen zu Überführen von regenerierten Adsorbtionsmitteln in das erste Fließbett und Vorrichtungen zum Einleiten der daraus entweichenden Gase in das zweite Fließbett.

16. Vorrichtung zum Herstellen von niederen einwertigen oder zweiwertigen Alkoholen gemäß Anspruch 1 bis 5 und 9 bis 11, welche umfaßt:

a) einen ersten Fließbettreaktor, Leitungsvorrichtungen zum Einleiten von Reaktand in den ersten Fließbettreaktor, Leitungsvorrichtungen zum Leiten von Gas aus dem ersten Reaktor in einen ersten Zyklon, Leitungsvorrichtungen zum Zurückführen von Feststoffen aus dem ersten Zyklon in den ersten Reaktor, Leitungsvorrichtungen zum Leiten von Gas aus dem ersten Zyklon in einen zweiten Reaktor;

b) Leitungsvorrichtung zum Leiten von Dampf in den zweiten Reaktor und Leitungsvorrichtungen zum Leiten von umsetzten Gasen aus dem zweiten Reaktor in einen dritten Reaktor, welcher ein Fließbettreaktor ist;

c) Leitungsvorrichtungen zum Leiten von Gasen aus dem dritten Reaktor in einen zweiten Zyklon, Leitungsvorrichtung zum Zurückführen von Feststoffen aus dem zweiten Zyklon in den dritten Reaktor, Vorrichtungen zum Leiten von Gas aus dem zweiten Zyklon in einen Kondensator, und Vorrichtung zum Abziehen von Kondensat aus dem Kondensator; und

d) Leitungsvorrichtungen zum Leiten von Gas aus dem Kondensator in Kompressorvorrichtungen zum Rezirkulieren von komprimiertem Gas zu den Leitungsvorrichtungen zum Einleiten von Reaktand in den ersten Fließbettreaktor.

17. Vorrichtung gemäß Anspruch 16, welche weiterhin Leitungsvorrichtungen zum Leiten von verbrauchtem Reaktanden aus dem ersten Fließbettreaktor in einen vierten Reaktor, welcher ebenfalls ein Fließbettreaktor ist, Leitungsvorrichtungen zum Einleiten von Gas in den vierten Reaktor und Leitungsvorrichtungen zum Leiten von regeneriertem Reagenz aus dem vierten Reaktor zurück zu dem ersten Fließbettreaktor umfaßt.

18. Vorrichtung gemäß Anspruch 16 oder 17, welche weiterhin einen fünften Reaktor enthält, welcher ebenfalls ein Fließbettreaktor ist, Leitungsvorrichtungen zum Leiten von verbrauchtem Fließbettmaterial aus dem dritten Reaktor in den fünften Reaktor, Leitungsvorrichtungen zum Einleiten von Gas in den fünften Reaktor, und Leitungsvorrichtungen zum Leiten von regeneriertem Fließbettmaterial zurück in den dritten Reaktor.

19. Vorrichtung gemäß einem der Ansprüche 16 bis 18, worin: der erste Fließbettreaktor ein Fließbett gemäß einem der Ansprüche 9 bis 11 enthält.

## Revendications

1. Procédé de production d'un alcool monobasique ou dibasique inférieur ayant de 1 à 4 atomes de carbone, qui comprend les étapes suivantes :

   a) la réaction d'une matière de départ appropriée avec un halogénure métallique, dans lequel le métal se trouve dans le plus élevé des deux états de valence possibles, pour former un produit réactionnel, un halogénure de métal correspondant ayant le métal dans le plus bas des deux états de valence possibles, et de l'acide halohydrique, et
   b) la réaction dudit produit réactionnel de l'étape (a) et de l'acide halohydrique, avec de l'oxyde de magnésium pour former l'alcanol monobasique ou dibasique inférieur correspondant et de l'hydrate d'halogénure de magnésium; et
   dans lequel la matière de départ pour la formation d'un alcool monobasique inférieur est un alcane inférieur ayant de 1 à 4 atomes de carbone, à partir duquel l'alcanol inférieur correspondant est obtenu; et la matière de départ pour la formation d'un alcool dibasique inférieur est soit un alcanol inférieur ayant de 1 à 4 atomes de carbone, soit un alcène inférieur ayant de 1 à 4 atomes de carbone, à partir duquel le glycol inférieur correspondant est obtenu.

2. Procédé selon la revendication 1 qui comprend en outre :

   c) la réaction de l'halogénure de métal avec l'acide halohydrique et de l'oxygène pour former un halogénure métallique; et
   d) la conversion de l'hydrate d'halogénure de magnésium en oxyde de magnésium et acide halohydrique.

3. Procédé selon la revendication 1 ou 2 dans lequel l'halogénure de métal est le chlorure de cuivre.

4. Procédé selon l'une quelconque des revendications précédentes mis en oeuvre dans une large mesure dans un lit fluidisé.

5. Procédé de production de méthanol à partir de méthane, qui consiste à :

   a) faire réagir du méthane avec un chlorure métallique, dans lequel le métal se trouve dans le plus élevé des deux états de valence possibles, pour former du chlorure de méthyle, le chlorure de métal correspondant ayant le métal dans le plus bas des deux états de valence possibles, et de l'acide chlorhydrique;

b) faire passer le chlorure de méthyle et l'acide chlorhydrique obtenus à partir de l'étape (a), ainsi que de la vapeur, sur un catalyseur composé de zéolite de magnésium pour former de l'alcool méthylique et de l'acide chlorhydrique;

c) faire réagir l'alcool méthylique et l'acide chlorhydrique obtenus à partir de l'étape (b) avec de l'oxyde de magnésium pour obtenir de l'alcool méthylique et de l'hydrate de chlorure de magnésium; et

d) convertir l'hydrate de chlorure de magnésium en oxyde de magnésium et acide chlorhydrique.

6. Procédé selon la revendication 5 dans lequel le chlorure de métal est le chlorure de cuivre.

7. Procédé selon la revendication 5 ou 6 mis en oeuvre dans une large mesure dans un lit fluidisé.

8. Procédé selon l'une quelconque des revendications 5 à 7 qui comprend en outre les étapes consistant à :

a) faire réagir du méthane avec du chlorure cuivrique dans un lit fluidisé comprenant un mélange d'oxyde de magnésium, de chlorure cuivrique et de chlorure cuivreux pour former une combinaison de gaz comprenant de l'acide chlorhydrique, du chlorure de méthyle et du méthane n'ayant pas réagi;

b) faire passer la combinaison des gaz et de la vapeur sur un catalyseur composé de zéolite de magnésium pour obtenir un mélange d'alcool méthylique, d'acide chlorhydrique et de méthane;

c) faire passer le mélange sur un lit fluidisé contenant de l'oxyde de magnésium, qui adsorbe la totalité de l'acide chlorhydrique;

d) faire se condenser l'alcool méthylique à partir de la combinaison restante d'alcool méthylique et de méthane; et

e) recycler le méthane dans l'étape (a).

9. Procédé selon la revendication 4, 7 ou 8 dans lequel le lit fluidisé comprend un mélange de chlorure cuivrique, de chlorure cuivreux et d'oxyde de magnésium.

10. Procédé selon la revendication 9 dans lequel le mélange comprend les composants spécifiés dans un rapport molaire approximatif de chlorure cuivrique/chlorure cuivreux/oxyde de magnésium de 1/0,1/2.

11. Procédé selon la revendication 9 ou 10 dans lequel le mélange comprend en outre de l'oxyde cuivrique.

12. Dispositif pour la production de méthanol à partir de méthane de la façon définie dans les revendications 6 à 8 et 9 à 11 qui comporte :

a) un réacteur à lit fluidisé comprenant, conune réactif, le lit fluidisé selon la revendication 9, des moyens pour introduire le méthane dans le réacteur et dans le lit fluidisé contenu dans ce réacteur, et des moyens de type conduite pour diriger le méthane n'ayant pas réagi et les gaz produits, de celui-ci jusqu'à (b);

b) un réacteur contenant un catalyseur, des moyens pour introduire de la vapeur dans les moyens de type conduite, des moyens pour faire passer le mélange résultant à travers le catalyseur contenu dans ce réacteur, et des seconds moyens de type conduite pour diriger le méthane n'ayant pas réagi et les produits réactionnels, de celui-ci jusqu'à (c);

c) un premier fluidiseur comprenant des moyens adsorbants pour adsorber l'acide chlorhydrique, et des troisièmes moyens de type conduite pour diriger les composants gazeux restants, dudit fluidiseur jusqu'à (d); et

d) des moyens de type condenseur pour faire se condenser le méthanol, et des quatrièmes moyens de type conduite pour renvoyer le méthanol n'ayant pas réagi vers le réacteur à lit fluidisé (a).

13. Dispositif selon la revendication 12, comportant en outre :

e) un second fluidiseur, des moyens pour amener le réactif usé du réacteur à lit fluidisé jusqu'au second fluidiseur, des moyens pour diriger de l'air et de l'acide chlorhydrique dans ledit second fluidiseur et à travers le réactif usé contenu à l'intérieur de ce second fluidiseur pour régénérer ledit réactif usé, des moyens pour amener le réactif régénéré jusqu'audit réacteur à lit fluidisé, et des cinquièmes moyens de type conduite pour amener les gaz issus du second fluidiseur jusqu'à (f); et

f) un troisième fluidiseur qui comprend des moyens adsorbants pour adsorber toutes les traces d'acide chlorhydrique dans les gaz retirés du second fluidiseur, et des moyens pour évacuer un gaz propre.

**14.** Dispositif selon la revendication 12 ou 13 comportant en outre :

g) un quatrième fluidiseur, des moyens pour amener les moyens absorbants usés du troisième fluidiseur jusqu'au quatrième fluidiseur, des moyens pour amener de l'air dans le quatrième fluidiseur et à travers les moyens adsorbants usés contenus dans ce quatrième fluidiseur pour régénérer lesdits moyens absorbants usés, des moyens pour acheminer les moyens adsorbants régénérés jusqu'au troisième fluidiseur, et des moyens pour amener les gaz effluents jusqu'au second fluidiseur.

**15.** Dispositif selon les revendications 12 à 14 comportant en outre :

h) un cinquième fluidiseur, des moyens pour acheminer les moyens adsorbants usés du premier fluidiseur jusqu'au cinquième fluidiseur, des moyens pour amener de l'air dans ledit cinquième fluidiseur et à travers les moyens adsorbants usés contenus à l'intérieur de ce cinquième fluidiseur pour régénérer lesdits moyens adsorbants usés, des moyens pour acheminer les moyens adsorbants régénérés jusqu'au premier fluidiseur, et des moyens pour amener les gaz émanant de celui-ci jusqu'au second fluidiseur.

**16.** Dispositif pour la production d'un alcool monobasique ou dibasique inférieur de la façon définie dans les revendications 1 à 5 et 9 à 11 qui comporte :

a) un premier réacteur à lit fluidisé, des moyens de type conduite pour introduire le réactif dans le premier réacteur à lit fluidisé, des moyens de type conduite pour amener le gaz issu dudit premier réacteur jusqu'à un premier cyclone, des moyens de type conduite pour renvoyer les solides issus du premier cyclone dans le premier réacteur, et des moyens de type conduite pour amener le gaz issu du premier cyclone jusqu'à un second réacteur;

b) des moyens de type conduite pour amener de la vapeur dans le second réacteur, et des moyens de type conduite pour amener les gaz ayant réagi issus du second réacteur jusqu'à un troisième réacteur, qui est un réacteur à lit fluidisé;

c) des moyens de type conduite pour amener le gaz issu du troisième réacteur jusqu'à un second cyclone, des moyens de type conduite pour renvoyer les solides issus du second cyclone jusqu'au troisième réacteur, des moyens pour amener le gaz issu du second cyclone jusqu'à' un condenseur, et des moyens pour soutirer le condensat du condenseur; et

d) des moyens de type conduite pour amener le gaz du condenseur jusqu'à des moyens de type compresseur pour faire recirculer le gaz comprimé jusqu'aux moyens de type conduite pour introduire le réactif dans le premier réacteur à lit fluidisé.

**17.** Dispositif selon la revendication 16 qui comporte en outre des moyens de type conduite permettant d'amener le réactif usé du premier réacteur à lit fluidisé jusqu'à un quatrième réacteur, qui est également un réacteur à lit fluidisé, des moyens de type conduite permettant l'introduction de gaz dans le quatrième réacteur, et des moyens de type conduite permettant de faire revenir le réactif régénéré issu du quatrième réacteur dans ledit premier réacteur à lit fluidisé.

**18.** Dispositif selon la revendication 16 ou 17 qui comporte en outre un cinquième réacteur, qui est également un réacteur à lit fluidisé, des moyens de type conduite pour amener la matière de lit fluidisé usée issue du troisième réacteur jusqu'au cinquième réacteur, des moyens de type conduite pour l'introduction de gaz dans le cinquième réacteur, et des moyens de type conduite pour faire revenir la matière de lit fluidisé régénérée dans ledit troisième réacteur.

**19.** Dispositif selon l'une quelconque des revendications 16 à 18 dans lequel :

le premier réacteur à lit fluidisé contient le lit fluidisé selon l'une quelconque des revendications 9 à 11.

# FIG. 1

EP 0 667 804 B1

FIGURE 2

EP 0 667 804 B1